# EUROPEAN PATENT SPECIFICATION

(11) **EP 3 013 376 B1**
(45) Date of publication and mention of the grant of the patent: **26.02.2020**
(21) Application number: 14742647.2
(22) Date of filing: 26.06.2014
(51) Int. Cl.: A61L 26/00, A61K 9/00, A61K 47/38, A61K 31/05

(54) **METHOD OF REDUCING SCARRING**
VERFAHREN ZUR VERMINDERUNG VON NARBENBILDUNG
PROCÉDÉ DE RÉDUCTION DE CICATRICES

(30) Priority: 26.06.2013 US 201361839769 P
(43) Date of publication of application: 04.05.2016
(73) Proprietor: Cole Reasearch & Design, LLC, Flowood, MS 39232 (US)
(72) Inventor: COLE, Jeptha, N., Flowood, MS 39232 (US)
(74) Representative: Titmus, Craig Edward
(86) International application number: PCT/US2014/044338
(87) International publication number: WO 2014/210308

(56) References cited:
- WO-A1-2012/129499
- US-A1- 2008 057 088
- US-A1- 2008 139 507
- DIONNE LORENA ET AL: "Normal scarring: importance of myofibroblasts", WOUND REPAIR AND REGENERATION, vol. 10, no. 2, 1 March 2002 (2002-03-01), pages 86-92, XP055144064, ISSN: 1067-1927, DOI: 10.1046/j.1524-475X.2002.00201.x
- ISMAIL YAMAN ET AL: "Effects of resveratrol on incisional wound healing in rats", SURGERY TODAY, vol. 43, no. 12, 15 December 2012 (2012-12-15), pages 1433-1438, XP055144076, ISSN: 0941-1291, DOI: 10.1007/s00595-012-0455-7
- KHANNA S ET AL: "Dermal Wound Healing Properties Of Redox-Active Grape Seed Proanthocyanidins", FREE RADICAL BIOLOGY AND MEDICINE, ELSEVIER INC, US, vol. 33, no. 8, 1 January 2002 (2002-01-01), pages 1089-1096, XP002518619, ISSN: 0891-5849, DOI: 10.1016/S0891-5849(02)00999-1

## Description

### BACKGROUND

Wound healing is a complex process, and involves the regulation of numerous cellular functions including the interactions of fibroblasts/fibrocytes, osteoblasts, chondrocytes, endothelial cells, inflammatory cells, epithelial cells and smooth muscle cells, with the extracellular matrix. Normal healing results in scar formation in humans. However, it is well known that certain animals, and even the human fetus, are capable of regenerative healing of wounds which is indistinguishable from surrounding skin.

Although the intricate details of wound healing are still being discovered, the process follows along a typical time line having four phases:
*Hemostasis Phase* - This phase includes vasoconstriction lasting for the first 5-10 minutes after the injury.
*Inflammation Phase* - This phase includes vasodilation and a cellular response by inflammatory macrophages, neutrophils and fibroblasts. Neutrophils undergo cannbilaization to produce transforming growth factor beta-1 (TGF-β1), which stimulates production of type I collagen (the mature collagen present in nomal skin) and stimulates fibroblast to myofibroblasts mediated by hyaluronic acid and epidermal growth factor receptor (EGFR). Bacteria, foreign particles and damaged cells are removed from the wound. Vasodilation starts at about 10 minutes after the initial injury, and the cellular response typically starts 30 minutes after the initial injury. Keratinocytes detach from the basement membrane and migrate to cover the exposed wound and connective tissue, and the wound clot is replaced with epithelial cells and granulation tissue (type III collagen). Differentiating keratinocytes also produce TGF-β1. The cellular response may last 7 to 8 days.
*Proliferation Phase* - This phase includes re-epithelialization of the wound, fibroplasia, including collagen synthesis and wound contraction. During this phase skin cells multiply and spread, covering the wound. Re-epithelialization typically starts 24 hours after the injury. Fibroplasia typically starts in 3 to 4 days after the injury. Myofibroblasts (present in granulation tissue) express alpha-smooth muscle actin and are responsible for wound contraction, which typically starts 7 days after the injury.
*Remodeling Phase* - This phase includes scar/collagen remodeling. The newly formed collagen matrix becomes cross linked and organized starting about 3 weeks from wound initiation and lasting as long as 1 year.

Scar formation is a typical response for normal healing in humans. As compared with normal skin, a scar contains an overproduction of type III and type I collagens, and the mixture is disorganized. The scar itself is not very elastic and is of a different color than normal skin. The scar is also missing the layer of kertinocytes found on normal skin. Furthermore, depending on how deep was the original wound, the scar may be missing the normal underlying layers of muscle, fat, blood vessels, and many layers of the skin; these missing layers may result in the scar forming a depression compared to the level of the surrounding skin.

Some animals are capable of scar free healing. In axolotls, there is a substantial reduction in neutrophil infiltration and a relatively long delay in production of new extracellular matrix during scar free healing. Studies with athymic nude mice indicate that up-regulation in metalloproteinase-9 (MMP-9) throughout the remodeling phase may contribute to scar free healing. Matrix metalloproteinases (MMP's) are a family of zinc dependent enzymes capable of degradation of extracellular matrix and are vital to the remodeling of the matrix and migration of cells. During normal human wound healing, MMP-9 degrades the type IV collagen of the basement membrane allowing keratinocytes to detach from the basement membrane and migrate to cover the exposed wound and connective tissue.

Human oral healing of wounds results in little to no scar formation. Oral mucosal wounds show a robust early up-regulation of MMP-1, MMP-2 and MMP-9 at 3 days after the initial injury, as compared to skin wounds at 14 days after the initial injury. The human fetus, which also shows scar free healing, is surrounded by amniotic fluid which contains high molecular weight hyaluronic acid. High molecular weight hyaluronic acid is known to increase expression of MMP-2 and MMP-9. Although high molecular weight hyaluronic acid application at a wound site can reduce scarring, a scar is nevertheless still formed.

Resveratrol (trans-3,4',5-trihydroxystilbene), a stilbenoid, is a grape polyphenol present in various plants, some food products, red wine and grapes. Resveratrol has anti-inflammatory, anti-carcinogenic and anti-oxidant properties, and has been extensively studied. Huge interest in resveratrol was created when it was discovered that it was able to activate the *SIRT1* gene, a gene implicated in the life span extension associated with calorie-restricted diets. However, resveratrol is poorly absorbed when consumed as a dietary supplement, and is subject to metabolic degradation, and beneficial effects have been difficult to observe in human clinical studies. US 2008/057088 discloses: a topical skin composition, useful for skin rejuvenation of dry, scarred, damaged or ageing skin, comprising Krebs cycle intermediates and an adjuvant for enhancing skin cell mitochondrial function, such as resveratrol. The compositions are able to reduce scars already present and resulting from surgery.

KHANNA S ET AL: "Dermal Wound Healing Properties Of Redox-Active Grape Seed Proanthocyanidins", FREE RADICAL BIOLOGY AND MEDICINE, ELSEVIER INC, US, vol. 33, no. 8, 1 January 2002 (2002-01-01), pages 1089-1096, discloses that topical resveratrol is useful in wound healing.

### SUMMARY

The scope of the invention is defined by the claims. Any references in the description to methods of treatment refer to the product of the present invention for use in a method of treatment.

The object of the present invention is the provision of a composition comprising resveratrol for use in reducing scar formation during wound healing, comprising applying said composition only into a wound which was formed at most one day before the applying, and in which no part of the skin surface of the wound is more than 3 cm from uninjured skin.

### BRIEF DESCRIPTION OF THE DRAWINGS

FIG. 1 is a photograph of a treated human patient's skin at the planned site of excision, prior to excision, from Example 3.
FIG. 2 is a photograph of a treated human patient's skin at the site of excision, 24 hours post-excision, from Example 3.
FIG. 3 is a photograph of an untreated human patient's skin at the site of excision, 24 hours post-excision, from Example 3.
FIG. 4 is a photograph of a treated human patient's skin at the site of excision, 36 hours post-excision, from Example 3.
FIG. 5 is a photograph of a treated human patient's skin at the site of excision, 72 hours post-excision, from Example 3.
FIG. 6 is a photograph of a treated human patient's skin at the site of excision, 96 hours post-excision, from Example 3.

### DETAILED DESCRIPTION

The present invention makes use of the discovery that compositions containing resveratrol, when applied into a wound soon after the initial injury, will greatly reduce scarring. In some cases, compositions containing resveratrol may even eliminate scarring altogether. Also discovered is that these effects of resveratrol may be enhanced when combined with one or more additional active agents.

It has been discovered that if a wound or incision is completely healed in less than 3 days, before fibroplasia begins, then no scar will be formed at the location of the wound or incision. Therefore composition containing resveratrol will allow for scar free healing when applied to wounds or incisions that do not have any injured or missing tissue which is more than 3 cm from uninjured tissue. Examples include almost all incisions purposefully created by a surgeon, because the surgeon is able to bring the edges of the skin at the location of the incision to well within 3 cm of each other. No part of the skin surface of the wound is more than 3 cm from uninjured skin, more preferably no part of the skin surface of the wound is more than 2 cm from uninjured skin, even more preferably no part of the skin surface of the wound is more than 1 cm from uninjured skin, and most preferably no part of the skin surface of the wound is more than 0.5 cm from uninjured skin.

Compositions containing resveratrol, either as the sole active agent or in combination with other active agents, is applied to a wound or incision at any time from prior to formation of a wound or incision up until at most one day after the formation of a wound or incision; more preferably prior to formation of a wound or incision, up until at most 1 hour after the formation of a wound or incision; and most preferably prior to formation of a wound or incision, up until at most 10 minutes after the formation of a wound or incision. Preferably, only a single application of a composition containing resveratrol is used. For example, a composition containing resveratrol may be applied topically to an incision site, or injected below an incision site, then the skin may be cut, optionally followed by closing the incision; for example the deep structures which have been cut under the skin may be tied down using VICRYL™ (polyglactin 910) sutures, and then skin sutured or sealed using DERMABOND ADVANCED™ topical skin adhesive or NEW-SKIN® liquid bandage. Alternatively, a composition containing resveratrol may be applied to the incision or wound after it is formed, followed by closing the wound or incision as described above.

Preferably, resveratrol is present in a composition at a concentration of at least 0.01 micromoles/liter, more preferably at a concentration of at least 0.10 micromoles/liter, and most preferably at a concentration of at least 0.50 micromoles/liter. Preferably, resveratrol is present in the composition at a concentration of at most 100 micromoles/liter, more preferably at most 40 micromoles/liter. Examples include 0.75, 0.80, 0.90, 1.0, 1.25, 1.5, 1.6, 1.7, 1.8, 1.9, 2.0, 2.1, 2.19, 2.2, 2.3, 2.4, 2.5, 2.6, 2.7, 2.8, 2.9, 3.0, 3.25, 3.5, 3.75, 4.0, 4.25, 4.5, 4.75, 5.0, 5.5, 6.0, 6.5, 7.0, 7.5, 8.0, 9.0, 10.0, 15.0, 20.0, 25.0, 30.0 and 35.0 micromoles/liter. Concentrations of resveratrol above 100 micromoles/liter appear to be cytotoxic to keratinocytes.

In some forms, such as gels and pastes, the delivery medium limits contact with the surrounding tissue, the surrounding tissue rapidly degrades the resveratrol, and the tissue itself will absorb the resveratrol, resulting in a much lower effective concentration of resveratrol. In those cases, the concentration of resveratrol may optionally be increased 10 fold. In those cases, preferably resveratrol is present in a composition at a concentration of at least 0. 1 micromoles/liter, more preferably at a concentration of at least 1.0 micromoles/liter, and most preferably at a concentration of at least 5.0 micromoles/liter. Preferably, resveratrol is present in those compositions at a concentration of at most 1000 micromoles/liter, more preferably at most 400 micromoles/liter. Examples include 7.5, 8.0, 9.0, 10, 12.5, 15, 16, 17, 18, 19, 20, 21, 21.9, 22, 23, 24, 25, 26, 27, 28, 29, 30, 32.5, 35, 37.5, 40, 42.5, 45, 47.5, 50, 55, 60, 65, 70, 75, 80, 90, 100, 150, 200, 250, 300 and 350 micromoles/liter.

Resveratrol has a very low solubility in water, however only that portion which is dissolved in water will exert its effects. Furthermore, if the resveratrol is applied dissolved in a hydrophobic medium, it may slowly diffuse into the surrounding aqueous medium, and undesirably extend the effective application time. Therefore, it is preferable that the resveratrol be applied as a solution in an aqueous medium. For ease of application in a clinical setting, preferably the aqueous medium is a gel, paste, foam, suspension or thickened solution. Examples include aqueous compositions containing hydroxypropyl methylcellulose, high molecular weight hyaluronic acid, polyethylene glycol, agar, dextrin, pectin, trehalose, xanthan gum, polyoxyethylene alkyl ethers, chitosan, guar gum and sodium alginate. Other vehicles, adjuvants and excipients, which are hydrophilic or have hydrophilic moieties, and are compatible which application into wounds, may also be used. Other pharmaceutically acceptable adjuvant, excipients and vehicles may also be included.

Premeasured amounts of the compositions containing resveratrol may also be used. These are referred to as unit dosage forms, since each premeasured amount is intended to be used on a single patient for one or more application, all used at the same time. Examples include prefilled syringes, pouches, packets and tubes. Another example would be a tube or dispenser which may be used to form foam of its contents just prior to application, for example by shaking or using a foaming agent. A self-foaming tablet, which forms foam when placed into water, could also be used. The volume of material present in these unit dosage forms may be at least 0.1 to 100 ml, or 1 to 50 ml. including 2, 3, 4, 5, 6, 7, 8, 9, 10, 11, 12, 13, 14, 15, 20, 25, 30, 35, 40 and 45 ml.

Other active agents may be included, such as other activators of *SIRT1;* HDAC2 (a class I histone deacetylase) inhibitors, such as trichostatin A; agents which stimulate the production of certain growth factors such as EGF, FGF-10 and IGF-1; luteolin; tretinoin (all-trans retinoic acid); and high molecular weight hyaluronic acid.

Although it is not known exactly how resveratrol reduces scarring, resveratrol does up-regulate and increase the expression of a variety of agents which are involved in wound healing. One possible explanation is that resveratrol causes the over-expression of MMP-9, interleukin-8 (IL-8) and SIRT1, and increases expression of EGFR on the keratinocyte membrane and nucleus. SIRT1 may then promote differentiation, motility and proliferation of keratinocytes, and deacetylation and inactivation of p53 protein thus inhibiting p53-dependent cell death from apoptosis in response to stress in human tenocytes (fibroblast-like tendon cells). SIRT1 may induce nitric oxide (NO) production, which inhibits class I HDAC 2 from blocking growth factors, including epithelial growth factor, keratinocyte growth factor 2, fibroblast growth factor 10 (FGF-10), and insulin-like growth factor 1 (IGF-1). SIRT1 may also decrease inflammation and apoptosis through a variety of mechanisms. IL-8 has a direct and profound stimulatory effect on the migration of keratinocytes, which is likely due via the PLC-gamma pathway, and furthermore IL-8 may recruit neutrophils. As noted above, MMP-9 degrades the type IV collagen of the basement membrane. EGFR may cause keratinocyte and fibroblast migration and may protect and repair tissue through nuclear DNA repair. Resveratrol may also inhibit NF-kB dependent proinflammatory and matrix degrading gene products induced by IL-1β and nicotinamide.

### EXAMPLES

### Example 1 (Study 101): In vivo application of compositions containing varying amounts of resveratrol in a rat model

In this example, 15 Sprague Dawley Rats, 6-8 weeks old, were placed randomly into 5 different groups, Study Groups 1-5, resulting in 3 animals per study group. An incision, 2 cm in length, was made on both the right and left shoulder of each rat: the left side was an untreated control, while the right side was treated with the Compositions 1-5, QID x 7 days. The Study Group number corresponding to the Composition number. The compositions contain the following amounts of resveratrol in a bacitracin/zinc ointment: 300 mg/g; 200 mg/g; 50 mg/g; 400 mg/g and 100 mg/g, respectively. Photographs were taken of both wounds at 1 week and 4 weeks. Prior to euthanizing the animals at 30 day, serum was obtained and analyzed for absorption of resveratrol. Punch biopsies were obtained from one rat per group at 30 days, one from the control incision and one from the treated incision.

Results and conclusions: Wound healing occurred rapidly, within 24-48 hours. At day 4 mild redness and irritation began to develop in all groups, with wound dehiscence with higher concentrations of resveratrol. Lower resveratrol concentrations were more effective.

Rapid early wound healing occurs with topical resveratrol application. High concentrations of resveratrol are cytotoxic to keratinocytes. Prolonged application of resveratrol does not improve wound healing and causes delayed wound healing.

### Example 2 (Study 102): In vivo application of various compositions containing resveratrol in a rat model

In this example, 10 Sprague Dawley Rats, 6-8 weeks old, were placed randomly into 5 different groups, Study Groups 1-5, resulting in 2 animals per study group. An incision, 2 cm in length, was made on both the right and left shoulder of each rat: the left side was an untreated control, while the right side was treated with the Compositions 1-5, with the Study Group number corresponding to the Composition number.

The constant ingredient in each composition is resveratrol and the concentration was kept constant at 400 microM in suspension in hydroxyproply methylcellulose (HPMC) 8% gel. Additional active ingredients including luteolin, Hhgh molecular weight hyaluronic acid, and retinoic acid were added to a total of five exclusive compounds. These five Compositions were: (1) 400 microM resveratrol, (2) 400 microM resveratrol and high molecular weight hyaluronic acid, (3) 400microM resveratrol and 0.01% Retinoic Acid, (4) resveratrol powder 0.01g, and (5) 400microM resveratrol and luteolin.

After each incision is made, the resveratrol containing composition was applied to the right incision just prior to closure using interrupted 5-0 nylon sutures. The left incision was also closed using interrupted 5-0 nylon sutures. Each incision was photographed and measurements will be taken, each day for 7 days. On the 4^{th} day, serum blood samples will be taken for systemic absorption assay. On the 8^{th} day, a punch biopsy will be taken from each test and control incision.

### Results and conclusions:

Gross morphologic observations during the eight days revealed some significant differences. Gross healing observation findings were graded subjectively by the primary researcher based on: A. Degree of epithelialization where 0%= no epithelialization over wound; 100% = full epithelial coverage over wound. B. Degree of erythema of skin surrounding wound where 0=no erythema; 1=slight erythema; 2=mild erythema; 3=moderate erythema; 4=moderate severe erythema; 5=severe erythema. Tabulated gross morphologic observation data is presented in the following table, where T = treated, C = control, and a numerical value of 180-189 was used to identify each rat:

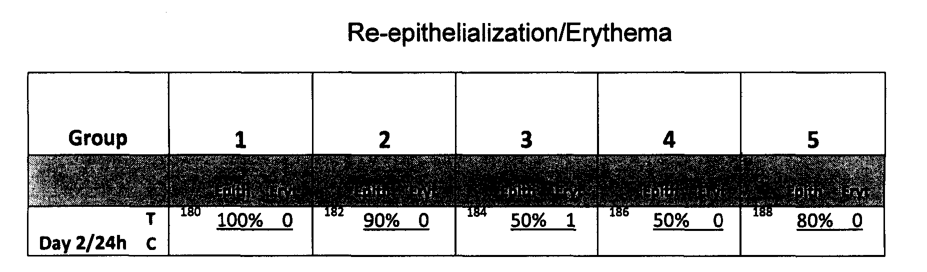

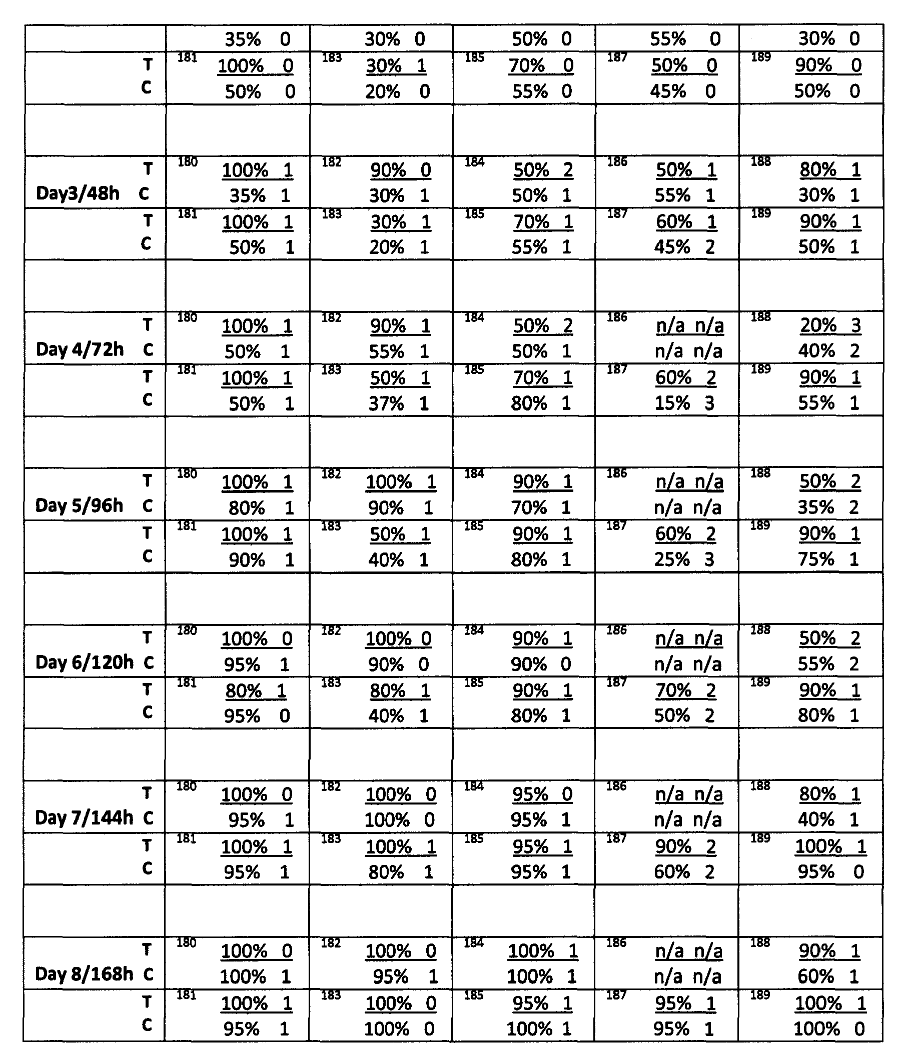

Re-epithelialization was defined by gross visual keratinocyte/skin spanning the incision and measured as a percentage of total wound length closure. The average time for total re-epithelialization for the control sites from each group was day 8 (168 hours). Group 1 (resveratrol) had rapid re-epithelialization in under 24 hours for one specimen, and in 96 hours in another specimen for an average of 60 hours. Group 2 (resveratrol and hyaluronic acid) had complete re-epithelialization noted at 96 hours. Group 3 (resveratrol and retinoic acid) had complete re-epithelialization at 168 hours (day 8). Group 4 (resveratrol powder 0.001 g) had not completed re-epithelialization by 168 hours (day 8). Group 5 (resveratrol and luteolin) had complete re-epithelialization in half of the specimens by day 7 and the remainder had not completed re-epithelialization by 168 hours (day 8).

Erythema was noted increased over all specimen's control sites starting at 48 hours and lasting until the end of the experiment at 168 hours (day 8). There was a slight decrease in control site erythema in some specimens by day 7. Group 1 had a noted decrease in erythema compared with control sites starting day 6 (120 hours) in one specimen, with the remaining specimen showing no difference from control site erythema. Group 2 had noted increase in treatment site erythema over control site starting on Day 3 (48 hours) to Day 4 (72 hours). Group 4 revealed no significant difference in erythema but had scratching injury to half the specimens resulting in increased erythema to both treatment and control sites. Group 5 revealed no significant erythema but again had scratching injury with both treatment and control site erythema in half the specimens.

Microscopic findings were graded subjectively by pathologist on a scale of 0 to 4 according to the intensity and extent of change, where 0= finding not present; 1=minimal; 2=mild; 3=moderate; and 4=marked. Histological exam revealed mild differences between treatment and control sites. All treatment sites except Group 3 treatment site were noted to have trichogranulomas from implantation of hair fragments at the time of wound creation. Group 1 revealed dermal treatment site mild (2/4) increase in mononuclear cell infiltrates over control (1/4). There was mild (2/4) increase in epidermal hyperkeratosis over control site (1/4) and a mild (2/4) increase in mononuclear cell infiltrates in the panniculus over control (1/4). Again, a trichogranuloma was noted in treatment site subcutis that was not noted in control site. Group 2 noted a decrease in epithelial hyperplasia in the treatment site, minimal (1/4), over control mild (2/4). Group 3 revealed mild (2/4) increase in epidermal hyperplasia and hyperkeratosis over control site minimal (1/4). Group 4 indicated a marked (4/4) increase in dermal mononuclear cell infiltrates over control mild (2/4). A dermal neutrophil infiltrate and epidermal crusting was noted in the treatment site of Group 4 and not noted in other specimens. Group 5 noted a mild (2/4) increase in treatment site dermal mononuclear cell infiltrates over control minimal (1/4).

Serum levels of resveratrol taken prior to euthanasia reveal an average blood serum level of 8.49 ng/ml.

Resveratrol within suspension in a non-toxic hydroxyproplylmethylcellulose gel exhibited rapid re-epithelialization at 400 microM concentration consistent with rapid up-regulation of MMP-9 and degredation of type IV collagen allowing keratinocyte migration. The other compounds containing retinoic acid, luteolin and powdered resveratrol revealed some improvement over control site healing but not as significant as resveratrol in HPMC gel. Some mild increase in erythema was noted at 48 hours with the resveratrol in HPMC gel, which could be due to keratinocyte toxicity. Resveratrol has been noted to be cytotoxic to keratinocytes over 100 microM effective concentration. The 400 microM concentration was used due to limited diffusion into surrounding tissue allowed by the gel and considering Fick's diffusion in a biological model.

Histology results indicate a limited decrease in monocyte chemoattracting protein 1 (MCP-1) as noted in prior studies, noted significantly in resveratrol in HPMC gel. Persistent monocyte migration was noted throughout treatment and control site incisions and hair introduction into the wound causing granulomas which complicated results. Although the resveratrol was applied through the depth of the incision, did not result in healing of all layers, and did not prevent depression and some superficial scar formation at the incision site.

In conclusion resveratrol in HPMC gel suspension caused accelerated keratinocyte migration and wound closure consistent with earlier study results.

### Example 3 (Study 103): In vivo application of various compositions containing resveratrol in a human trial

In this human trial, a total of 8 human subjects were treated, separated into 5 groups: Groups 1-3 and 5 having a single subject, and Group 4 having 4 subjects. Each group was treated with the respectively number compositions, each containing resveratrol in an 8% hydroxypropylmethylcellulose base: (1) 800 microM resveratrol, (2) 400 microM resveratrol, (3) 1600 microM resveratrol, (4) 40 microM resveratrol, and (5) control (no resveratrol).

Consent was obtained from each patient, and pre-surgical photographs were taken. The incisions were prepared using a sterile prep and drape, and an existing scar or mole excised. Hemostasis was carried out, and then the appropriate composition was applied. A subcuticular stitching was used on each incision, using vicryl sutures. A dressing was applied for 24 hours. Post-surgical photographs were taken. Each patient took photographs of their incision each day for 4 day, with a 1 week follow-up appointment.

Results and conclusions: All resveratrol containing compositions resulted in decreased wound closure time, by delivering rapid cross-linking and remodeling of the human surgical wounds. The resveratrol containing compositions inhibited factors that contribute to scarring and produced dramatically reduced scarring, compared to the control composition, in this study. A potential benefit of the resveratrol compositions would be reduced antibiotic use due to early wound closure and a reduction of open would size as a source of infection. In addition to reducing scarring from surgical wounds, the resveratrol containing compositions could also be used to reduce scarring from acne, stretch marks, burns, and during the excision of existing scars.

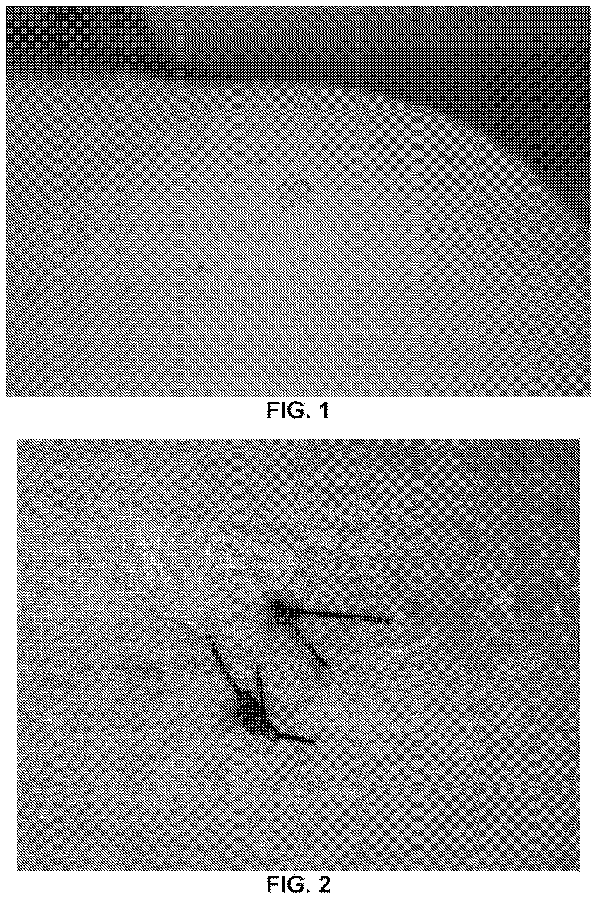

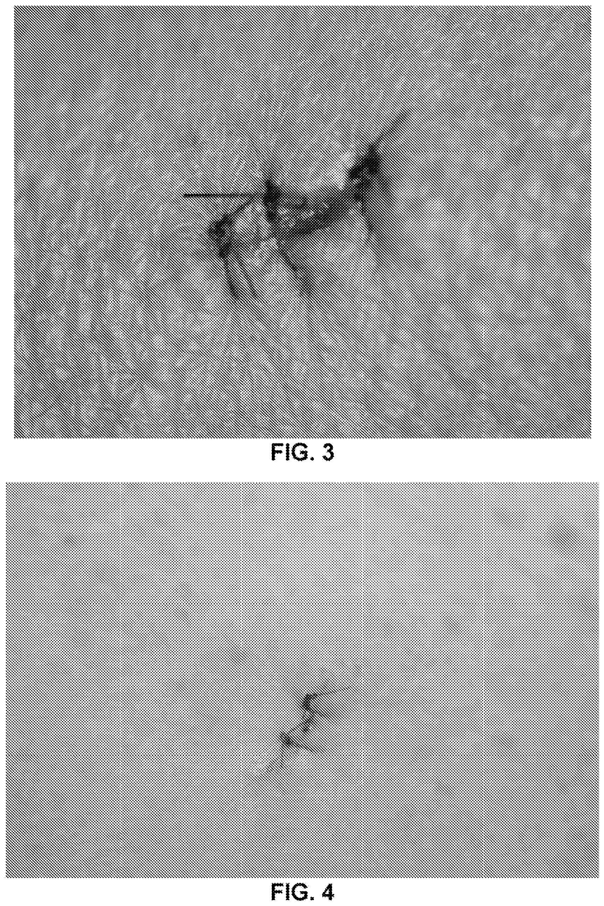

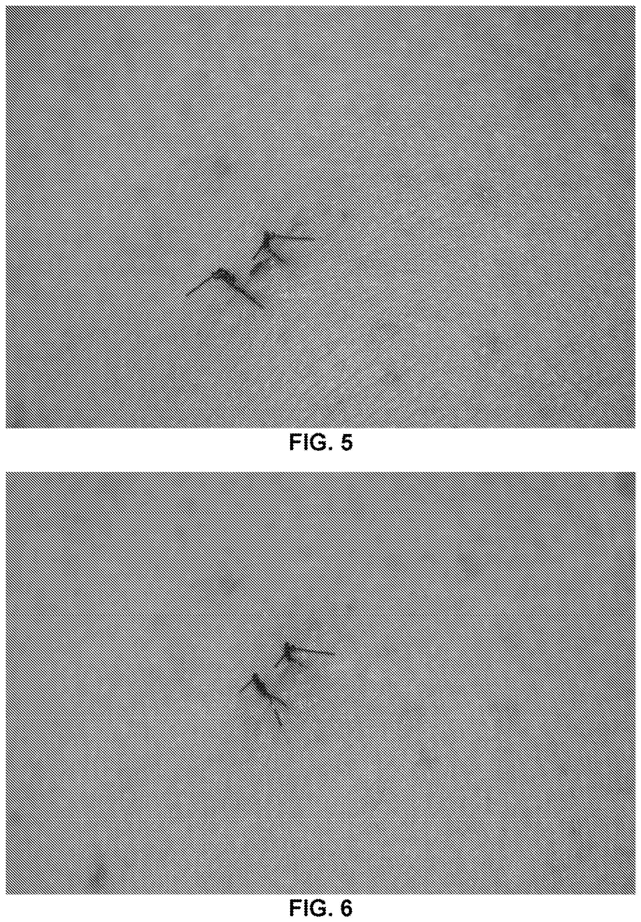

Exemplary photos of treated excisions, prior to excision and at 24, 36, 72 and 96 hours post-excision, and an untreated excision at 24 hours post-excision, are shown in FIG. 1-6.

### REFERENCES

Ehrlich H, Krummel T: Regulation of wound healing from a connective tissue perspective. Wound Repair & Regeneration 1996, 4(2):203-210.
Leung A, Crombleholme TM, Keswani SG: Fetal wound healing: implictions for minimal scar formation. Curr Opin Pediatr 2012, Jun24(3): 371-8.
Manuel J, Gawronska-Kozak B: Matrix metalloproteinase 9 (MMP-9) is upregulated during scarless wound healing in athymic nude mice. Matrix Biology 2006, 25:505-514.
Seifert AW, Monaghan J, Voss S, Maden M: Skin regernation in adult axolotls: a blueprint for scar-free healing in vertebrates. PLoS One 2012, 7: 4
Polette M, Nawrocki-Raby B, Gilles C, Clavell C, Birembaut P: Tumor invasion and matrix metalloproteinases. Crit.Rev. Oncol Hematol 2004, 49:179-186.
Salo T, Makela M, Kylmaniemi M, Autio-Harmainen H, Larjava H: Expression of matrix metalloproteinase-2 and -9 during early human wound healing. Lab Invest 1994, Feb;70(2):176-82.
Giannelis,G: Matrix metalloproteinases in scarless wound healing. Electronic Theses and Dissertations 2008 to 2011, July. (Available at hdl.handle.net/2429/36241).
Guo MS, Wu YY, Liang ZB: Hyaluronic acid increases MMP-2 and MMP-9 expressions in cultured trabecular meshwork cells from patients with primary open-angle glaucoma. Mol Vis 2012,18:11175-81.
Ndiaye M, Philippe C, Mukhtar H, Ahmad N: The grape antioxidant resveratrol for skin disorders: promise, prospects, and challenges. Arch Biochem Biophys2011, Apr 15:508(2): 164-70.
Gweon E, Kim S: Resveratrol induces MMP-9 and cell migration via the p38 kinase and PI-3K pathways in HT1080 human fibrosarcoma cells. Oncol Rep 2013, Feb 29(2): 826-34.
Ghosh S, Liu B, Zhou Z: Resveratrol activates SIRT1 in a Lamin A-dependent manner. Cell Cycle 2013 Mar 15;12(6):872-6.
Blander G, Bhimavarapu A, Mammone T, Maes D, Elliston K, Reich C, Matsui MS, Buarente L, Loureiro JJ. SIRT1 promotes differentiation of normal human keratinocytes. J Invest Dermatol 2009 Jan;129(1):41-9.
Thompson NL, Flander KC, Smith JM, Ellingsworth LR, Roberts AB, Sporn MB. Expressions of transforming growth factor-beta 1 in specific cells and tissues of adult and neonatal mice. J Cell Biol. 1989:108:661-9.
Midgley A, Rogers M, Hallett M, Clayton A, Bowen T, Phillips A, Steadman R.Transforming growth factor-beta 1 (TGF-B1)-stimulated fibroblast to myofibroblast differentiation is mediated by hyaluronan (HA)-facilitated epidermal growth factor receptor (EGFR) and CD44 colocalisation in lipid rafts. J Biol Chem 2013 Apr 15 [Epub ahead of print].
Busch F, Mobashieri A, Shayan P, Stahlmann R, Shakibaei M. Sirt-1 Is Required for the Inhibition of Apoptosis and Inflammatory Responses in Human Tenocytes. J Biol Chem 2012 Jul 27; 287(31):25770-25781.
Spallotta F, Cencioni C, Straino S, Nanni S, Rosati J, Artuso S, Manni I Colussi C, Piaggio G, Martelli F, Valent S, Mai A, Caposgrassi MD, Faretti A, Gaetano C. A Nitric Oxide-dependent Cross-talk between Clas I and II Histone Deacetylases Accelerates Skin Repari. J Bio Chem. 2013 Apr 19; 288(16):11004-12.
Pastore S, Lulli D, Maurelli R, Dellambra E, DeLuca C, Korkina LG; Resveratrol induces long-lasting IL-8 expression and peculiar EGFR activation/distributio in human keratinocytes:mechanisms and implications for skin administration. PLoS One 2013:8(3):e59632.
Jiang WG, Sanders AJ, Ruge F, Harding KG. Influence of interleukin-8(IL-8) and IL-8 receptors on the migration of human keratinocytes, the role of PLC-gamma and potential clinical implications. Exp The Med 2012 Feb; 3(2):231-236.
Steiger S, Harper JL. Neutrophil cannibalism triggers transforming growth factor beta1 production and self regulation of neutrophil inflammatory function in monosodium urate monohydrate crystal-induced inflammation in mice. Arthritis Rheum 2013 Mar: 65(3):815-23.
Holian O, Walter RJ. Resveratrol inhibits the proliferation of normal human keratinocytes in vitro. J Cell Biochem Supp12001; Suppl 36:55-62.
Kim JJ, Kim SJ, Kim SY, Park SH, Kim EC. The role of SIRT1 on angiogenic and odontogenic potential in human dental pulp cells. J Endod 2012 Jul; 38(7):899-906.
Williams LD, et al.; Safety studies conducted on high-purity transresveratrol in experimental animals. Food Chem Toxicol, 2009 Sept; 47(9):2170-82.
Polonini HC, et al. Photoprotective activity of resveratrol analogues. Bioorg Med Chem, 2013 Feb 15; 21(4):964-8.
Hung CF, Lin YK, Huang ZR, Fang JY. Delivery of resveratrol, a red wine polyphenol, from solutions and hydrogels via the skin. Biol Pharm Bull. 2008 May; 31(5):955-62.
Alonso C, Martí M, Martinez V, Rubio L, Parra JL, Coderch L. Antioxidant cosmeto-textiles: skin assessment. Eur J Pharm Biopharm. 2013 May;84(1):192-9. doi: 10.1016/j.ejpb.2012.12.004. Epub 2012 Dec 20.
Machesney M, Tidman N, Waseem A, Kirby L, Leigh I. Activated keratinocytes in the epidermis of hypertrophic scars. Am J Pathol. 1998 May; 152(5): 1133-41.
Fagone E, Conte E, Gili E, Fruciano M, Pistorio MP, Lo Furno D, Giuffrida R, Crimi N, Vancheri C. Resveratrol inhibits transforming growth factor-β-induced proliferation and differentiation of ex vivo human lung fibroblasts into myofibroblasts through ERK/Akt inhibition and PTEN restoration. Exp Lung Res. 2011 Apr; 37(3):162-74. doi: 10.3109/01902148.2010.524722. Epub 2011 Jan 26.
Sheu SY, Chen WS, Sun JS, Lin FH, Wu T. Biological characterization of oxidized hyaluronic acid/resveratrol hydrogel for cartilage tissue engineering. J Biomed Mater Res A. 2013 Apr 18. doi: 10.1002/jbm.a.34653. [Epub ahead of print]
Fearmonti R, Bond J, Erdmann D, Levinson H. A review of scar scales and scar measuring devices. Eplasty. 2010 Jun 21; 10:e43.
Busch F, Mobasheri A, Shayan P, Stahlmann R, Shakibaei M. Sirt-1 is required for the inhibition of apoptosis and inflammatory responses in human tenocytes. J Biol Chem. 2012 Jul 27; 287(31):25770-81. doi: 10.1074/jbc.M112.355420. Epub 2012 Jun 11.
Nayor D, Kiefer D. Living longer, Healthier Lives with Resveratrol. Le Magazine 2008 Feb. (available at www.lef.org).
Resveratrol. en.wikipedia.org/wiki/Resveratrol (downloaded 6-21-2013).
Amato *et al.* U.S. Pat. Pub., Publication no. US 2011/0245345 (Oct. 6, 2011).
McKay *et al.* U.S. Pat. Pub., Publication no. US 2011/0038965 (Feb. 17, 2011).
NEW-SKIN® Cover. Protect. Prevent. newskinproducts.com/products.aspx (downloaded 6-20-2013).

## Claims

1. A composition comprising resveratrol for use in reducing scar formation during wound healing, comprising applying said composition only into a wound which was formed at most one day before the applying, and in which no part of the skin surface of the wound is more than 3 cm from uninjured skin.

2. The composition for use according to claim 1, wherein an amount of resveratrol present in the composition is
a) 0.01 to 1000 micromoles/liter;
b) 0.1 to 1000 micromoles/liter; or
c) 0.5 to 1000 micromoles/liter.

3. The composition for use according to claim 1 or claim 2, wherein an amount of resveratrol present in the composition is:
a) 0.01 to 400 micromoles/liter
b) 0.1 to 400 micromoles/liter;
c) 0.5 to 400 micromoles/liter;
d) 0.01 to 200 micromoles/liter;
e) 0.1 to 200 micromoles/liter;
f) 0.5 to 200 micromoles/liter;
g) 0.01 to 100 micromoles/liter;
h) 0.1 to 100 micromoles/liter; or
i) 0.5 to 100 micromoles/liter.

4. The composition for use according to any of the preceding claims, wherein the wound was formed at most one hour before the applying; optionally wherein the wound was formed at most 10 minutes before the applying.

5. The composition for use according to any of the preceding claims, wherein no part of the skin surface of the wound is more than 2 cm from uninjured skin; optionally wherein no part of the skin surface of the wound is more than 1 cm from uninjured skin; optionally wherein no part of the skin surface of the wound is more than 0.5 cm from uninjured skin.

6. The composition for use according to any of the preceding claims, wherein the composition is a gel, paste, foam, suspension or thickened solution.

7. The composition for use according to any of the preceding claims, wherein the composition is a gel or thickened solution.

8. The composition for use according to any of the preceding claims, wherein the composition further comprises at least one member selected from the group consisting of hydroxypropyl methylcellulose, high molecular weight hyaluronic acid, polyethylene glycol, agar, dextrin, pectin, trehalose, xanthan gum, polyoxyethylene alkyl ethers, chitosan, guar gum and sodium alginate.

9. The composition for use according to any of the preceding claims, wherein the composition further comprises at least one member selected from the group consisting of hydroxypropyl methylcellulose and high molecular weight hyaluronic acid.

10. The composition for use according to any of the preceding claims, wherein the composition further comprises hydroxypropyl methylcellulose.

11. The composition for use according to any of the preceding claims, wherein the composition is provided as a unit dosage form; optionally wherein:
(a) the unit dosage form has a volume of 0.1 to 100 ml; or
(b) the unit dosage form has a volume of 1 to 50 ml; or
(c) the unit dosage form has a volume of 5 to 10 ml.

12. The composition for use according to any of the preceding claims, wherein the unit dosage form is selected from the group consisting of a prefilled syringe, a pouch, a packet and a tube.

13. The composition for use according to any of the preceding claims, wherein the composition further comprises at least one member selected from the group consisting of a *SIRT1* activator; a HDAC2 inhibitor; an agent which stimulates the production of EGF, FGF-10 or IGF-1; luteolin; tretinoin; and high molecular weight hyaluronic acid.

14. The composition for use according to any of the preceding claims, wherein the wound is an incision.

15. The composition for use according to any of the preceding claims, wherein said reducing scar formation during wound healing comprises applying said composition into said wound in a single application only.

## Patentansprüche

1. Zusammensetzung umfassend Resveratrol zur Verwendung bei der Verminderung der Narbenbildung während der Wundheilung, umfassend die Applikation der genannten Zusammensetzung nur in eine Wunde, die höchstens einen Tag vor der Applikation herbeigeführt wurde, und in der kein Teil der Hautoberfläche der Wunde mehr als 3 cm von unverletzter Haut ist.

2. Zusammensetzung zur Verwendung nach Anspruch 1, wobei eine in der Zusammensetzung vorliegende Menge an Resveratrol
(a) 0,01 bis 1000 Mikromol/Liter;
(b) 0,1 bis 1000 Mikromol/Liter; oder
(c) 0,5 bis 1000 Mikromol/Liter beträgt.

3. Zusammensetzung zur Verwendung nach Anspruch 1 oder Anspruch 2, wobei eine in der Zusammensetzung vorliegende Menge an Resveratrol
(a) 0,01 bis 400 Mikromol/Liter;
(b) 0,1 bis 400 Mikromol/Liter;
(c) 0,5 bis 400 Mikromol/Liter;
(d) 0,01 bis 200 Mikromol/Liter;
(e) 0,1 bis 200 Mikromol/Liter;
(f) 0,5 bis 200 Mikromol/Liter;
(g) 0,01 bis 100 Mikromol/Liter;
(h) 0,1 bis 100 Mikromol/Liter; oder
(i) 0,5 bis 100 Mikromol/Liter beträgt.

4. Zusammensetzung zur Verwendung nach einem der vorangehenden Ansprüche, wobei die Wunde höchstens eine Stunde vor der Applikation herbeigeführt wurde; gegebenenfalls, wobei die Wunde höchstens 10 Minuten vor der Applikation herbeigeführt wurde.

5. Zusammensetzung zur Verwendung nach einem der vorangehenden Ansprüche, wobei kein Teil der Hautoberfläche der Wunde mehr als 2 cm von unverletzter Haut ist; gegebenenfalls, wobei kein Teil der Hautoberfläche der Wunde mehr als 1 cm von unverletzter Haut ist; gegebenenfalls, wobei kein Teil der Hautoberfläche der Wunde mehr als 0,5 cm von unverletzter Haut ist.

6. Zusammensetzung zur Verwendung nach einem der vorangehenden Ansprüche, wobei die Zusammensetzung ein Gel, eine Paste, ein Schaum, eine Suspension oder eine eingedickte Lösung ist.

7. Zusammensetzung zur Verwendung nach einem der vorangehenden Ansprüche, wobei die Zusammensetzung ein Gel oder eine eingedickte Lösung ist.

8. Zusammensetzung zur Verwendung nach einem der vorangehenden Ansprüche, wobei die Zusammensetzung weiter mindestens ein Glied umfasst, das aus der Gruppe ausgewählt ist, bestehend aus Hydroxypropylmethylcellulose, hochmolekularer Hyaluronsäure, Polyethylenglykol, Agar, Dextrin, Pektin, Trehalose, Xanthangummi, Polyoxyethylenalkylethern, Chitosan, Guargummi und Natriumalginat.

9. Zusammensetzung zur Verwendung nach einem der vorangehenden Ansprüche, wobei die Zusammensetzung weiter mindestens ein Glied umfasst, das aus der Gruppe ausgewählt ist, bestehend aus Hydroxypropylmethylcellulose und hochmolekularer Hyaluronsäure.

10. Zusammensetzung zur Verwendung nach einem der vorangehenden Ansprüche, wobei die Zusammensetzung weiter Hydroxypropylmethylcellulose umfasst.

11. Zusammensetzung zur Verwendung nach einem der vorangehenden Ansprüche, wobei die Zusammensetzung als eine Einzeldosierungsform vorgesehen ist; gegebenenfalls wobei:
(a) die Einzeldosierungsform ein Volumen von 0,1 ml bis 100 ml aufweist; oder
(b) die Einzeldosierungsform ein Volumen von 1 ml bis 50 ml aufweist; oder
(c) die Einzeldosierungsform ein Volumen von 5 ml bis 10 ml aufweist.

12. Zusammensetzung zur Verwendung nach einem der vorangehenden Ansprüche, wobei die Einzeldosierungsform aus der Gruppe ausgewählt ist, bestehend aus einer vorgefüllten Spritze, einem Beutel, einer Packung und einer Tube.

13. Zusammensetzung zur Verwendung nach einem der vorangehenden Ansprüche, wobei die Zusammensetzung weiter mindestens ein Glied umfasst, das aus der Gruppe ausgewählt ist, bestehend aus einem SIRT1-Aktivator; einem HDAC2-Inhibitor; einem die Produktion von EGF, FGF-10 oder IGF-1 stimulierenden Mittel; Luteolin; Tretinoin; und hochmolekularer Hyaluronsäure.

14. Zusammensetzung zur Verwendung nach einem der vorangehenden Ansprüche, wobei die Wunde eine Inzision ist.

15. Zusammensetzung zur Verwendung nach einem der vorangehenden Ansprüche, wobei die genannte Verminderung der Narbenbildung während der Wundheilung die Applikation der genannten Zusammensetzung in die genannte Wunde in nur einer einzelnen Applikation umfasst.

## Revendications

1. Composition comprenant du resvératrol destinée à une utilisation dans la réduction de la formation de cicatrices lors de la cicatrisation de plaies, comprenant l'application de ladite composition seulement dans une plaie qui a été formée au maximum un jour avant l'application, et aucune partie de la surface de la peau de la plaie n'étant située à plus de 3 cm de la peau non lésée.

2. Composition destinée à une utilisation selon la revendication 1, une quantité suivante de resvératrol étant présente dans la composition :
a) 0,01 à 1 000 micromoles/litre ;
b) 0,1 à 1 000 micromoles/litre ; ou
c) 0,5 à 1 000 micromoles/litre.

3. Composition destinée à une utilisation selon la revendication 1 ou la revendication 2, une quantité suivante de resvératrol étant présente dans la composition :
a) 0,01 à 400 micromoles/litre ;
b) 0,1 à 400 micromoles/litre ;
c) 0,5 à 400 micromoles/litre ;
d) 0,01 à 200 micromoles/litre ;
e) 0,1 à 200 micromoles/litre ;
f) 0,5 à 200 micromoles/litre ;
g) 0,01 à 100 micromoles/litre ;
h) 0,1 à 100 micromoles/litre ; ou
i) 0,5 à 100 micromoles/litre.

4. Composition destinée à une utilisation selon l'une quelconque des revendications précédentes, la plaie ayant été formée au maximum une heure avant l'application ; optionnellement, la plaie ayant été formée au maximum 10 minutes avant l'application.

5. Composition destinée à une utilisation selon l'une quelconque des revendications précédentes, aucune partie de la surface de la peau de la plaie ne se trouvant à plus de 2 cm de la peau non lésée ; optionnellement, aucune partie de la surface de la peau de la plaie ne se trouvant à plus de 1 cm de la peau non lésée ; optionnellement, aucune partie de la surface de la peau de la plaie ne se trouvant à plus de 0,5 cm de la peau non lésée.

6. Composition destinée à une utilisation selon l'une quelconque des revendications précédentes, la composition étant un gel, une pâte, une mousse, une suspension ou une solution épaissie.

7. Composition destinée à une utilisation selon l'une quelconque des revendications précédentes, la composition étant un gel ou une solution épaissie.

8. Composition destinée à une utilisation selon l'une quelconque des revendications précédentes, la composition comprenant en outre au moins un élément sélectionné dans le groupe constitué de l'hydroxypropylméthylcellulose, de l'acide hyaluronique à haute masse moléculaire, du polyéthylène glycol, de la gélose, de la dextrine, de la pectine, du tréhalose, de la gomme xanthane, d'alkyléthers de polyoxyéthylène, du chitosane, de la gomme de guar et de l'alginate de sodium.

9. Composition destinée à une utilisation selon l'une quelconque des revendications précédentes, la composition comprenant en outre au moins un élément sélectionné dans le groupe constitué de l'hydroxypropylméthylcellulose et de l'acide hyaluronique à haute masse moléculaire.

10. Composition destinée à une utilisation selon l'une quelconque des revendications précédentes, la composition comprenant en outre de l'hydroxypropylméthylcellulose.

11. Composition destinée à une utilisation selon l'une quelconque des revendications précédentes, la composition étant fournie comme une forme galénique unitaire ; optionnellement :
a) la forme galénique unitaire ayant un volume de 0,1 à 100 ml ; ou
b) la forme galénique unitaire ayant un volume de 1 à 50 ml ; ou
c) la forme galénique unitaire ayant un volume de 5 à 10 ml.

12. Composition destinée à une utilisation selon l'une quelconque des revendications précédentes, la forme galénique unitaire étant sélectionnée dans le groupe constitué d'une seringue préremplie, d'une poche, d'un sachet et d'un tube.

13. Composition destinée à une utilisation selon l'une quelconque des revendications précédentes, la composition comprenant en outre au moins un élément sélectionné dans le groupe constitué d'un activateur de *SIRT1,* d'un inhibiteur de HDAC2, d'un agent qui stimule la production d'EGF, de FGF-10 ou d'IGF-1, de la lutéoline, de la trétinoïne, et de l'acide hyaluronique à haute masse moléculaire.

14. Composition destinée à une utilisation selon l'une quelconque des revendications précédentes, la plaie étant une incision.

15. Composition destinée à une utilisation selon l'une quelconque des revendications précédentes, ladite réduction de la formation de cicatrices lors de la cicatrisation de plaies comprenant l'application de ladite composition dans ladite plaie en une seule application seulement.
